# EUROPEAN PATENT APPLICATION

(11) **EP 4 464 291 A1**
(43) Date of publication of application: **20.11.2024**
(21) Application number: 24193942.0
(22) Date of filing: 16.03.2023
(51) Int. Cl.: A61F 13/02

(54) **WOUND DRESSING**

(30) Priority: 16.03.2022 GB 202203625
(62) Divisional of application: 23713741.9
(71) Applicant: ConvaTec Limited, Deeside, Flintshire CH5 2NU (GB)
(72) Inventor: STEVEN, Jade, Deeside, CH5 2NU (GB); SHAW, Helen, Deeside, CH5 2NU (GB); KESTEVEN, Donna, Deeside, CH5 2NU (GB); BREWER, Claire, Deeside, CH5 2NU (GB); BALLAMY, Lucy, Deeside, CH5 2NU (GB)
(74) Representative: Wilson Gunn

(57) **Abstract**

Disclosed is a wound dressing comprising: a backing layer; a wound contact layer having a first surface for directly contacting a wound bed and a second surface facing the backing layer, the wound contact layer comprising gel forming fibers; and, a superabsorbent layer, wherein the superabsorbent layer is located between the backing layer and the second surface of the wound contact layer.

## Description

### Technical Field of the Invention

The present invention relates to an absorbent wound dressing.

### Background to the Invention

It is known to make wound dressings for use on exuding wounds. Such dressings manage the exudate produced by the wound in a variety of ways. For example, some dressings, mainly those based on foam, manage exudate by absorbing the exudate and allowing the moisture taken up by the dressing to evaporate through the backing or top of the dressing. Such dressings are not designed to absorb and retain the exudate but to absorb and expel the exudate by moisture vapour transmission so that they maximise the level of exudate handled within the limitations of their design. A disadvantage of such dressings is that the lateral spread of the exudate across the dressing is not contained because of the nature of the open structure of the foam and this can cause normal skin surrounding the wound to become macerated as the whole of the dressing surface becomes saturated.

A further disadvantage of the open structure of the foam is that when the dressing is put under pressure, for example when compression is applied or when force is applied to the dressing due to the patient sitting, lying or rolling over, the exudaite can be squeezed out of the porous, open foam structure and into contact with the wound and/or surrounding skin surfaces creating the potential for peri-wound maceration and, in the case of chronic wounds, further wound breakdown due to damage caused by certain components of chronic wound exudate. A further disadvantage of such dressings is that rapid loss of exudate by evaporation can cause the wound surface to become desiccated over time which impedes healing.

Other dressings, mainly those based on absorbents that gel, manage exudate by absorbing it and retaining it within the dressing. The moisture in the absorbent can still be lost by vapour transmission from the dressing, but less readily than with a foam absorbent because the exudate is retained and held within the gelled absorbent. As the absorbent is acting as a reservoir for exudate, it needs to have sufficient capacity to retain exudate throughout the wear time of the dressing. This affects the quantity of absorbent needed in the wound dressing which of course affects the thickness and conformability of the dressing overall. In addition, in general with gelling absorbents, particularly fibrous gelling absorbents, the relationship between the thickness of the gelling absorbent layer or its weight per unit area and its absorbency is not linear. This means that a careful balance needs to be struck between absorbency, conformability and moisture vapour transmission.

EP2498829 describes wound dressing which discloses an absorbent component for a wound dressing, the absorbent component comprising a wound contacting layer comprising gel forming fibres bound to a foam layer. The combination of a wound contact layer which absorbs exudate by gelling so that lateral spread of the exudate is contained, with a foam layer which absorbs exudate but readily releases it through moisture vapour transmission has shown an effective way of increasing fluid handling capability. Further, the absorbent wound contacting layer helps to control the fluid handling properties by limiting the lateral spread of exudate in the foam and increasing its exudate retention compared to the use of foam alone. In turn the moisture vapour transmission properties of the wound contacting layer may be improved.

Gelling absorbents are particularly suited to moderately exuding wounds. This because, at low exudate levels, the wound bad may become dehydrated and adhere to the dressing, and at high exudate levels, the fluid handling capability of the gelling wound contact layer may not be sufficient.

Known wound dressings may include an adhesive contact layer (which may be referred to as a wound-site adhesive layer) which adheres the wound dressing to the patient's skin. Known adhesive contact layers may be provided as a continuous layer which extends fully across the wound dressing and includes perforations, or an adhesive contact layer provided at the border of the dressing only and which may or may not have perforations. An example of a wound dressing having a perforated adhesive contact layer is provided in US 10231874. This document discloses a wound contact layer comprising a perforated polyurethane film that is coated with a skin-compatible adhesive. The perforations are disclosed as being any desirable shape and having a size range of 0.025mm to 1.2mm which is considered small enough to help prevent tissue ingrowth into the wound dressing while allowing wound exudate to flow into the dressing. Other dressings which include a perforated adhesive layer include the applicant's existing product Aquacel^{®} Form Pro product. This product includes a perforated wound site adhesion layer with 1.5mm diameter perforations to increase breathability of the dressing.

The present invention seeks to provide an improved absorbent wound dressing.

### Summary of the Invention

The present invention provides a wound dressing according to the appended claims.

In a first aspect, there is disclosed herein a wound dressing comprising: a backing layer; a wound contact layer; and, a wound-site adhesive layer for adhering the wound dressing to a wound site. The wound contact layer may comprise a first surface for directly contacting a wound bed and a second surface facing the backing layer. The wound-site adhesive layer may be perforated and comprise a plurality of perforations.

The adhesive layer may comprise a border region which surrounds the wound contact layer. The perforations may have a diameter between 1.6mm and 2.7mm. In some embodiments, the perforations may preferably comprise a diameter between 2.1mm and 2.3mm, more preferably 2.2mm.

The adhesive layer may be restricted to the border region which surrounds the wound contact layer. In some embodiments, the adhesive layer may partially overlap the wound contact layer. The adhesive layer may adhere to the wound contact layer so as to hold the wound contact layer in a fixed relation to the backing layer and/or the wound site.

In some embodiments, the adhesive layer may extend continuously across the first surface of the wound contact layer. Hence, the contact between the wound contact layer and wound may be through the perforations.

The perforations may be uniformly distributed across the full extent of the adhesive layer. In some embodiments, the perforations in the border region may differ from the perforations in a central region which corresponds to the wound contact layer. The perforations between the central region and border region may differ in size, shape, distribution (i.e. pattern) or number. The perforations in the border region may be uniform. The perforations in the central region may be uniform. The perforations may have a common size. The common size may be a common diameter. Hence, the diameter of the perforations may all be 2.2mm, for example.

The perforations may be any desired shape. The shape may be common to all of the perforations. The perforations may be round, for example, circular or oval.

The perforations are arranged in a regular array comprising rows and columns. The spacing between centers of the columns may be greater than the spacing between rows. Adjacent rows may be laterally offset from one another. The spacing between the columns may be between 3.9mm and 8.1mm. The spacing between the rows may be between 2.5mm and 5.1mm.

The adhesive may comprise a silicone adhesive. The adhesive layer may comprise an adhesive having a density of between 70g and 150g per square meter.

The adhesive layer may comprise an inner edge which defines a central window (which may be referred to as a central region) through which the wound contact layer may be exposed.

The wound contact layer may comprise an outer edge and may overlap the adhesive layer around the periphery of the wound contact layer so as to be adhered thereto. The width of the overlap between the inner edge of the adhesive layer and the outer edge of the wound contact layer may vary around the periphery of the wound contact layer.

The overlap may comprise one or more corner region and one or more edge region of the wound contact layer and adhesive layer. The width of the overlap in the corner regions may be greater than the overlap in an adjacent edge region.

The one or more corner region may comprise an inner edge having a first radius of curvature, and the outer edge comprises a second radius of curvature. The first radius of curvature may be greater than the second radius of curvature.

The backing layer may be adhered to the adhesive layer. The wound dressing may further comprise a superabsorbent layer. The superabsorbent layer may be in adhesive-free contact with the backing layer.

The superabsorbent layer may comprise a first surface facing the wound contact layer and a second surface facing the backing layer. The superabsorbent layer first surface may comprise an adhesive layer.

In a second aspect there is disclosed herein a wound dressing comprising: a backing layer; a wound contact layer having a first surface for directly contacting a wound bed and a second surface facing the backing layer, and a superabsorbent layer.

The wound contact layer may comprise gel forming fibers. The superabsorbent layer may be located between the backing layer and the second surface of the wound contact layer.

The superabsorbent layer may have an outer profile and the wound contact layer may have a corresponding outer profile which is in register with the outer profile of the superabsorbent layer. In other words, the outer profiles of the superabsorbent layer and wound contact layer may be the same and totally aligned.

Accordingly, in an embodiment, there is provided a wound dressing comprising: a backing layer; a wound contact layer having a first surface for directly contacting a wound bed and a second surface facing the backing layer, the wound contact layer comprising gel forming fibers; and, a superabsorbent layer, wherein the superabsorbent layer is located between the backing layer and the second surface of the wound contact layer, wherein the superabsorbent layer has an outer profile and the wound contact layer has a corresponding outer profile which is in register with the outer profile of the superabsorbent layer.

The wound dressing may further comprise a foam layer located between the backing layer and the second surface of the wound contact layer. The foam layer may have a foam layer outer profile which corresponds with and is in register with the outer profile of the superabsorbent layer.

Accordingly, in an embodiment, there is provided a wound dressing comprising: a backing layer; a wound contact layer having a first surface for directly contacting a wound bed and a second surface facing the backing layer, the wound contact layer comprising gel forming fibers; and, a superabsorbent layer located between the backing layer and the second surface of the wound contact layer, wherein the superabsorbent layer has an outer profile and the wound contact layer has a corresponding outer profile which is in register with the outer profile of the superabsorbent layer, wherein the dressing comprises a foam layer located between the backing layer and the second surface of the wound contact layer, wherein the foam layer has a foam layer outer profile which corresponds with and is in register with the outer profile of the superabsorbent layer.

The foam layer may be located between the wound contact layer and the superabsorbent layer.

The superabsorbent layer may comprise fibers, optionally non-woven fibers. The superabsorbent layer may comprise non-woven fibers only. The non-woven fibers may be polyacrylate fibers.

Accordingly, in an embodiment, there is provided a wound dressing comprising: a backing layer; a wound contact layer having a first surface for directly contacting a wound bed and a second surface facing the backing layer, the wound contact layer comprising gel forming fibers; and, a superabsorbent layer located between the backing layer and the second surface of the wound contact layer, wherein the superabsorbent layer comprises fibers, wherein the superabsorbent layer comprises non-woven fibers only, wherein the non-woven fibers are polyacrylate fibers.

The superabsorbent layer may have a thickness of between 1.5mm and 2.5mm, optionally 2mm.

Accordingly, in an embodiment, there is provided a wound dressing comprising: a backing layer; a wound contact layer having a first surface for directly contacting a wound bed and a second surface facing the backing layer, the wound contact layer comprising gel forming fibers; and, a superabsorbent layer located between the backing layer and the second surface of the wound contact layer, wherein the superabsorbent layer has a thickness of between 1.5mm and 2.5mm, optionally 2mm.

The superabsorbent layer may comprise a first surface facing the wound contact layer and a second surface facing the backing layer. The superabsorbent layer first surface may comprise an intra-layer adhesive. The intra-layer adhesive may be a continual later, perforated layer, liquid or scatter coat adhesive for example. The intra-layer adhesive may be a web comprising a continuous or broken layer of adhesive which is inserted in between the respective adjacent layers and activated to bond the layers together. The intra-layer adhesive may be thermally activated for example in which heat and pressure are applied to during a lamination process. The intra-layer adhesive may comprise a web of adhesive strands or dots.

The super-absorbent layer second surface may be configured to be movable relative to the backing layer. The superabsorbent layer may contact the backing layer. The contact between the superabsorbent layer may allow for relative movement, e.g. sliding contact or differential expansive contact.

The backing layer may comprise an adhesive border region which surrounds a non-adhesive central region. The superabsorbent layer may be located fully within the non-adhesive central region such that the superabsorbent layer is not adhered to the backing layer.

The foam layer may comprise a first, wound facing surface and a second, backing layer facing surface. The foam layer first surface may comprise a foam first adhesive layer. The second surface of the foam layer may comprise a foam second adhesive layer. The foam layer and superabsorbent layer may be adhered together.

The foam layer may comprise a polyurethane foam. The foam layer may comprise an aliphatic foam or a methylene diphenyl disoocyanate foam.

The foam layer may have a thickness of between 1.3mm and 3.2mm, optionally 2.5mm.

The wound contact layer may comprise a cellulose fiber. The wound contact layer may comprise carboxymethylated cellulose fibers. The wound contact layer may have a thickness between 1mm and 1.5mm. The wound contact layer may have a base weight of between 70gsm and 150gsm, optionally 70gsm.

The backing layer may comprise a polyurethane material. The backing layer may have a thickness of 30 microns.

The wound contact layer may be unfenestrated. In other words, the wound contact layer may lack fenestrations.

The dressing may further comprise a wound-site adhesive layer for adhering the wound dressing at a wound site, wherein the adhesive layer comprises a border region which surrounds the wound contact layer, wherein the adhesive layer may comprise perforations, the perforations may have a diameter of between 1.6mm and 2.7mm, optionally 2.2mm.

Accordingly, in an embodiment, there is provided a wound dressing comprising: a backing layer; a wound contact layer having a first surface for directly contacting a wound bed and a second surface facing the backing layer, the wound contact layer comprising gel forming fibers; and, a superabsorbent layer located between the backing layer and the second surface of the wound contact layer, wherein the dressing further comprises a wound-site adhesive layer for adhering the wound dressing at a wound site, wherein the adhesive layer comprises a border region which surrounds the wound contact layer, wherein the adhesive layer comprises perforations, the perforations having a diameter of between 1.6mm and 2.7mm, optionally 2.2mm.

In a third aspect, there is disclosed herein a wound dressing comprising: a wound contact layer; a backing layer; an intermediate layer located between the backing layer and wound contact layer. The wound contact layer may comprise a first surface facing the wound contact layer and a second surface facing the backing layer.

The first surface of the intermediate layer may be bonded directly or indirectly to the wound contact layer. The second surface may be unbonded to the backing layer.

The dressing may further comprise a wound-site adhesive layer provided on a peripheral region of the backing layer for adhering the backing layer to a wound site.

The intermediate layer may be adhered directly or indirectly to the wound contact layer.

The wound dressing may further comprise an adhesive to provide the adherence of the intermediate layer to the wound contact layer. The adhesive may be referred to as an intra-layer adhesive or a binder layer. The adhesive may comprise a scatter coat adhesive.

The intermediate layer may comprise a superabsorbent layer.

The wound dressing may further comprise a foam layer located between the wound contact layer and backing layer. The foam layer may be located between the wound contact layer and the first surface of the superabsorbent layer. The foam layer may be adhered to the wound contact layer.

The wound-site adhesive layer may comprise a border region of adhesive which surrounds a central region in which the wound contact layer is located. The border region may partially cover the wound contact layer. The adhesive layer may comprise an inner edge defining a central window through which the wound contact layer is exposed.

The skilled person will appreciate that, except where mutually exclusive, a feature described in relation to any one of the aspects, embodiments or examples of the present disclosure may be applied mutatis mutandis to any other aspect, embodiment or example of the present disclosure. Furthermore, except where mutually exclusive, any feature described herein may be applied to any aspect and/or combined with any other feature described herein.

### Brief Description of the Drawings

In order that the invention may be more clearly understood one or more embodiments thereof will now be described, by way of example only, with reference to the accompanying drawings, of which:
Figure 1 shows an exploded view of a wound dressing according to an embodiment of the present disclosure;
Figure 2 shows an exploded view of wound dressing according to an alternative embodiment of the present invention; and,
Figure 3 shows a perforation pattern of a wound-site adhesive layer.
Figure 4 shows a further example of a wound dressing.
Figure 5 shows an example of a wound dressing in plan view.

### Detailed Description of the Invention

In the following detailed description, numerous specific details are set forth in order to provide a thorough understanding of various embodiments and the inventive concept. However, those skilled in the art will understand that: the present invention may be practiced without these specific details or with known equivalents of these specific details; that the present invention is not limited to the described embodiments; and, that the present invention may be practiced in a variety of alternative embodiments. It will also be appreciated that well known methods, procedures, components, and systems may have not been described in detail.

The wound dressings described herein are absorbent wound dressings having a plurality of layers for absorbing exudate from a wound. The wound dressings may comprise a backing layer, a super-absorbency layer, a wound contact layer, and a wound-site adhesive layer. In some embodiments, the dressing may also comprise a transmission layer. However, the layers or combination of layers described herein may comprise inventive aspects in their own right which may be used independently of the other layers which are described in combination herein. Thus, for example, the perforated wound-site adhesive layer may or may not be utilised in a dressing having a super-absorbency layer. In other examples, a dressing having the super-absorbency layer may be used in the absence of a wound-site adhesive layer or a wound-site adhesive layer having perforations. Nonetheless, providing a dressing having a backing layer, a super-absorbency layer, a wound contact layer, and a perforated wound-site adhesive layer may be particularly advantageous with the various components providing a synergistic benefit for the performance of the dressing in terms of adhesion, (e.g. wear time), absorbency and versatility.

In addition to the above layers, the wound dressings described herein and in accordance with embodiments of the disclosure may additionally include one or more additional layers, such as a foam layer, and/or an intralayer adhesive layer, for example. The additional layers may be provided to increase the performance of the dressing in terms of one or more of: fluid absorbency, conformability, retention, percentage retention, and fluid handling capacity. The wound dressing may also incorporate a release layer which protects the adhesive layer prior to use and helps preserve the sterility of the wound dressing whilst the dressing is being applied to a wound.

The wound dressings are described as a number of layers which are generally provided in a stacked configuration in which the wound contact layer is arranged proximate to the wound in use and the backing layer is provided as an outer layer which defines an external surface of the dressing. The various layers are generally concentrically arranged in the present disclosure but this need not be the case and the layers may be asymmetrically stacked on top of each other in some embodiments.

Each of the layers may be described herein in relation to the backing layer and the wound site. Thus, each layer which is located between wound site and backing layer may comprise a first surface which faces inwardly and towards the wound site, and a second surface which faces outwardly from the wound site and towards the backing layer.

One or more of the layers may be provided only in specific regions. For example, the wound-site adhesive layer may be provided only in a peripheral or border region of the dressing such that a central region does not include the wound-site adhesive layer. Further, the some of the layers may be located solely within a central region of the dressing, thereby being inward of the peripheral edge.

In some embodiments, the wound dressing may comprise: a wound contact layer; a backing layer and an intermediate layer located between the backing layer and wound contact layer. The intermediate layer may comprise a first surface facing the wound contact layer and a second surface facing the backing layer. The first surface of the intermediate layer may be bonded directly or indirectly to the wound contact layer. The second surface may be bonded or unbonded to the backing layer so as to prevent or allow lateral movement therebetween and to help control moisture transmission through the backing layer.

The intermediate layer may comprise: a super-absorbency layer or a foam layer. The wound dressing may comprise a plurality of intermediate layers in which an uppermost first intermediate layer is provided in unbonded contact with the backing layer, and is indirectly bonded to the wound contact layer via a second intermediate layer. This may be advantageous when the backing layer is a superabsorbent layer, for example.

The bonding of the first intermediate layer to the lower layers of the wound dressing whilst providing an unbonded connection between the intermediate layer and the backing layer, allows for lateral movement between the intermediate layer and backing layer and relative differential expansion and/or contraction. Providing this freedom of movement reduces or prevents any interlayer stress with the backing layer and the potential for mechanical deformation, e.g. wrinkling, which may result during manufacture (for example during EtO sterilisation, especially if the backing layer is PU film and/or the adjacent intermediate layer is a superabsorbent layer) or in use of the dressing.

In some embodiments, the uppermost first intermediate layer may be provided in direct bonded contact with the backing layer. This may be advantageous when the first intermediate layer is a foam, for example.

The wound dressing may further comprise a wound-site adhesive layer provided on a peripheral region of the backing layer for adhering the backing layer to a wound site.

In some embodiments, the wound dressing may comprise: a wound contact layer; a backing layer and a plurality of intermediate layers located between the backing layer and wound contact layer. The intermediate layers may comprise a superabsorbent layer and, optionally, a foam layer. The plurality of intermediate layers may be attached together, for example, as a laminate. The laminated intermediate layers may be adhered together. The adhesive may comprise a scatter coat adhesive.

The inclusion of a foam layer may allow the dressing to have increased absorbency and may aid the later spread of fluid to help increase MTVR.

In some embodiments, the wound dressing may comprise: a backing layer; a wound contact layer having a first surface for directly contacting a wound bed and a second surface facing the backing layer. The wound contact layer may comprise gel forming fibres. The wound dressing may further comprise a superabsorbent layer. The superabsorbent layer may be located between the backing layer and the second surface of the wound contact layer.

Providing a super-absorbent layer with a gelling wound contact layer can increase the fluid absorption, retention, percentage retention and fluid handling capability of the wound dressing. In particular, the inclusion of a gelling wound contact layer maintains a moist wound healing environment allowing a greater extraction of fluid with the superabsorbent layer than in a conventional dressing having a superabsorbent layer.

In some embodiments, the wound dressing may comprise: a backing layer; a wound contact layer having a first surface for directly contacting a wound bed and a second surface facing the backing layer; and, a perforated wound-site adhesive layer for adhering the wound dressing to a wound site.

Providing perforations can increase adhesion of the wound dressing, improve absorbency, and increase the versatility of the dressing.

The adhesive layer may comprise a border region which surrounds the wound contact layer and may optionally extend across the entirety of the dressing. The perforations may have a diameter between 1.6mm and 2.7mm. Providing perforations of this size, which are generally much larger than those known in the prior art, provides an unexpected advantage when balancing adhesion and the rate of absorbency of the dressing. Generally, increasing the percentage of overall open area provided by the perforations increases the speed of absorbency whilst reducing adhesion.

The applicants have found that having perforations of approximately 2.2mm provides an exemplary perforation size which balances the speed of absorption with adhesion. The reducing the size of the perforations below 2.2mm increases the effects of surface tension, whilst increasing the size of perforation further reduces adhesion with little to no appreciable increase in the rate of absorption.

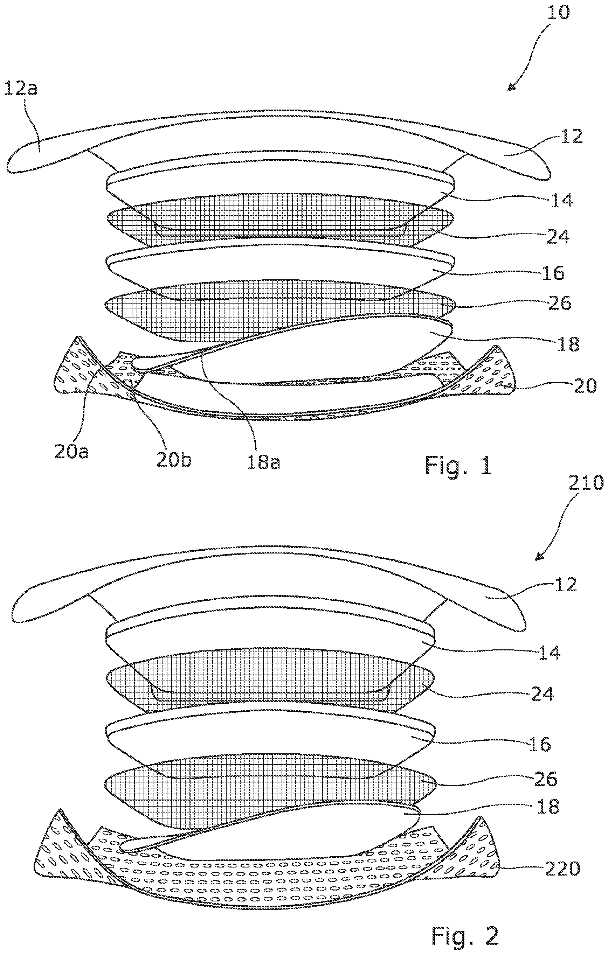

Further, providing a uniform distribution of perforations across the dressing may simplify the manufacturing process for the dressings. Hence, the size of the perforation may involve a compromise between the performance requirements of a border region of the dressing in which breathability is a dominant concern, and a wound contacting portion in which the interface of the dressing and the wound bed is of primary importance. Figure 1 shows an exploded view of a wound dressing 10 according to an embodiment of the present invention. As shown, the wound dressing 10 may comprise, in order: a backing layer 12, a super-absorbent layer 14, a foam layer 16, wound contact layer 18 and a wound-site adhesive layer 20. The order of the various layers may be interchanged in some embodiments such that, for example, the foam layer 16 and superabsorbent layer 14 may be switched to provide a wound dressing comprising, in top down order: a backing layer 12. a foam layer 16, a super-absorbent layer 14, a wound contact layer 18 and, wound-site adhesive layer 20. Further, although this disclosure is concerned primarily with wound dressings including a foam layer, it is contemplated that the some of the teachings disclosed herein may apply equally to embodiments in which the foam layer 16 is be omitted.

As shown, the superabsorbent layer 14 may have an outer profile and the wound contact layer 18 may have a corresponding outer profile which is in register with the outer profile of the superabsorbent layer 14. The foam layer 16 may have a foam layer outer profile which corresponds with and is in register with the outer profile of the superabsorbent layer 14. In addition, the wound contact layer 18 may be unfenestrated. In other words, the wound contact layer 18 may lack fenestrations.

Figure 2 shows an alternative arrangement in which a wound dressing 210 comprises: a backing layer 12, a super-absorbent layer 14, a foam layer 16, a wound contact layer 18 and a wound-site adhesive layer 220. As with Figure 1, the order of the various layers shown in Figure 2 may be interchanged in some embodiments and the foam layer 16 may be omitted.

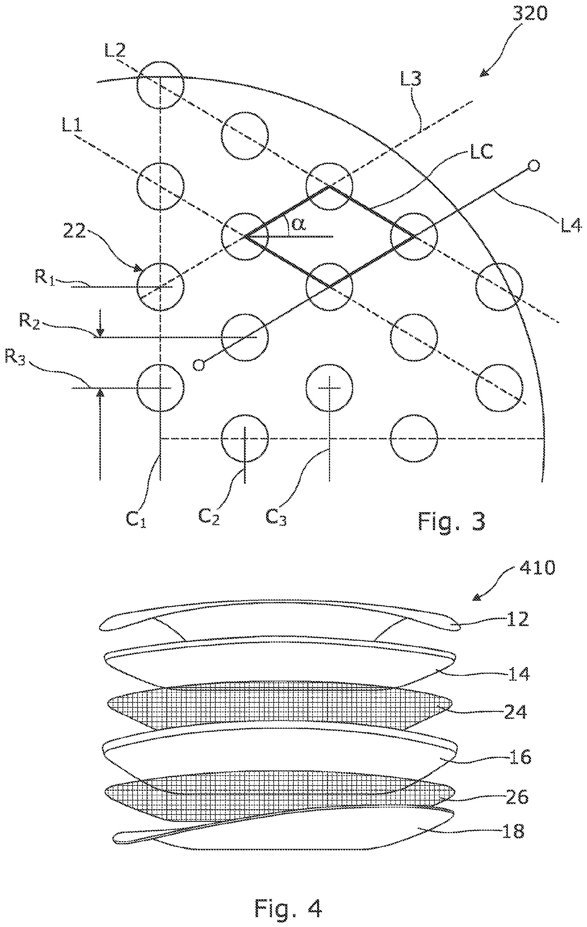

Figure 4 shows an alternative wound dressing 410 in which there is no wound-site adhesive layer. Here, the different layers share a common footprint and are typically used with an additional bandage or tape, such as a compression bandage, to locate it in place. Figure 4 additionally shows intra-layer adhesives 24, 26 which may be used in any of the dressings disclosed herein to adhere adjacent layers together.

As can be seen, a difference between the wound dressings shown in Figures 1 and 2 relates to the wound-site adhesive layer 22, 220. In the embodiment of Figure 1, the wound dressing 10 is provided with a peripheral border wound-site adhesive layer 22, whereas Figure 2 comprises a continuous wound-site adhesive layer 220 which extends across the full extent of the dressing and under lies the wound contact layer 18. The backing layer 12, super-absorbency layer 14, foam layer 16 and wound contact layer 18 may be similar for both of the embodiments of Figures 1 and 2. As such, the descriptions of each of the respective layers provided herein may be applicable to either or both wound dressings 10 and 210. It will also be appreciated that, as noted above, the position of the foam layer 16 and super-absorbency layer 14 may be interchanged in each dressing.

The wound-site adhesive layers 20, 220 shown in Figures 1 and 2 extend from the extreme peripheral edge 12a of the backing layer 12, and thus wound dressing 10, radially inwards towards the central region and wound contact layer 18. The outermost area of the wound-site adhesive layer 20, 220 provided in the border region contacts the backing layer 12 such that the backing layer 12 can be adhered directly to the patient around the wound site. As such, the backing layer 12 is adhered and sealed adjacent to the wound site so as to provide an envelope in which the other wound dressing layers are provided.

The wound-site adhesive layer 20 of Figure 1 comprises a radially outer edge 20a which coincides with the peripheral edge 12a of the backing layer 12, and extends inwards to terminate at an inner edge 20b which defines a central region of the dressing. The inner edge 20b may additionally lie radially inwards of a corresponding outer edge 18a of the wound contact layer 18 such that the two layers overlap when viewed in plan. Thus, the wound contact layer 18 may include an edge portion which contacts and is adhered to the wound-site adhesive layer 20 provided by the adhesive border. The overlap may be provided to securely locate the wound contact layer 18 in relation to the backing layer 12 and wound bed such that it is retained in-situ once the wound dressing 10 has been applied to the wound site. In contrast, the wound-site adhesive layer 220 of Figure 2 does not comprise a radially inner edge and extends fully across the extent of the wound contact layer 18. Nevertheless, the central region and border region may comprise different perforation sizes of patterns as described further below.

The choice as to whether to have the border only wound-site adhesive layer 20 or the full wound-site adhesive layer 220 may be application specific and determined by the exudate level of the wound being dressed. Thus, for dressings to be used in a low to medium exudate level, the full wound-site adhesive layer 220 may be chosen, and for dressings to be used in a higher exudate level, the border only wound-site adhesive layer 20 may be chosen to maximise the exudate absorption.

In wounds with high levels of exudate, the perforations may allow for movement of fluid through to the super-absorbent layer 14 while the use of a silicone adhesive layer 220 helps prevent wound adhesion to low exudate areas (it will be appreciated that wounds can have mixed aetiology). The dressings disclosed herein may be of particular use in pressure ulcer prevention and/or where skin protection around wounds is required.

In some dressings, the wound contact layer may be pre-hydrated with saline to donate fluid to wounds to support autolytic debridement.

The wound-site adhesive layer 20 may be any suitable pressure sensitive adhesive known in the art such as a hydrocolloid, polyurethane, rubber based adhesive, acrylic adhesive or silicone adhesive. Acrylic adhesives are known to provide reasonable adhesion human skin but are generally not repositionable. Silicone adhesives also provide a good adhesion to human skin but are hydrophobic and so provide a barrier to exudate and may limit to the moisture transmission vapour rate, MTVR. Hence, the use of perforations 22 with a silicone adhesive may be particularly advantageous. The silicone adhesive may be any suitable silicone adhesive such as Dow Corning 9900, Dow Corning 9800, Dow Corning 1020, or Wacker 2117, Wacker Silpuran 2114,. The base weight of the adhesive layer may be in a range of between 70gsm and 200gsm. For example, the base weight may be 70gsm, 80gsm, 100gsm, 130gsm, 150gsm, or 200gsm. The thickness of the adhesive may be between 20 microns and 40 microns. For example, the thickness may be 20 microns, 25 microns, 30 microns or 40 microns. In a preferred embodiment, the adhesive layer may have a coat thickness of approximately 25 microns and a coat weight of 100gsm.

The wound-site adhesive layers 20 and 220 of Figures 1 and 2 may include perforations 22. The perforations 22 may be provided to improve the performance of the dressing 10, 210. The improved performance may relate to the rate of exudate absorption, adhesion duration, i.e. wear time, for the dressing, and versatility of the dressing 10, 210, that is, the range of exudate levels the dressing may be used for.

In more detail, the wear time of the wound dressing may be increased by increasing the Trans Epidermal Water Loss, TEWL, in the border area where moisture build-up, e.g. sweat, can be relatively large thereby causing the dressing to fail. The perforations over the wound contact layer allow for exudate absorption through the perforations where it is then transferred to the outer layers to increase the Moisture Vapour Transmission Rate, MVTR, of the adhesive layer 20, 220.

As noted above, the embodiment of Figure 2 shows the perforations 22 extending across the full extent of the wound contact layer 18 so as to expose the wound contact layer 18 to the wound through the perforations 22. Providing the perforations 22 across the wound contact layer helps control the flow of exudate from the wound into the wound contact layer 18 and superabsorbent layer 14 (if present).

For example, if the rate of exudate is low or decreases, then the wound may become dehydrated and the wound contact layer 18 may become adhered to the wound. Thus, the use of perforations 22 under the wound contact layer 18 may reduce the contacting surface area with the wound. This, in combination with a suitable wound contact layer 18 and super-absorbent layer 14, may allow a larger volume of exudate to be extracted over an extended period of time. This issue may be reduced where a hydrophobic wound contact adhesive such as silicon is used.

Further, by selecting the correct size of perforation 22, it may be possible to ensure that the wound dressing 10, 210 is effective over a large range of exudate levels meaning a single dressing can be used on a broader range of wounds.

As noted above, the selection of the size of perforation may be complicated by the fact that there are multiple requirements for the perforations. In the case of the perforations 22 provided between the backing layer 12 and skin, there is a requirement to find a balance between breathability and adhesion to increase the wear time. In the case of the perforations 22 provided between the wound contact layer 18 and wound bed, there is a balance between fluid handling properties of the perforations 22 and maintaining the health of the wound site by preventing adhesion of the wound contact layer to the wound bed. To address this design conflict, in some wound dressings, the different areas of the wound dressing may be provided with different perforations to meet the varying requirements. However, providing different perforations in different areas requires a more complex manufacturing process for the wound-site adhesion layer.

The applicants have discovered that controlling the size of the perforation size of between 1.6mm and 2.7mm, which is considerably larger than the perforations disclosed in US 10231874 described briefly in the background section, provides a good balance between wear time and exudate absorption control for the dressing. More specifically, as noted above, the applicants have found a more preferred range of perforation is 2.1mm to 2.3mm, most preferably 2.2mm. It will be appreciated that the sizes of perforations 22 provided herein may be nominal and subject to normal manufacturing tolerances.

The perforations 22 are generally through-holes or apertures which extend through the thickness of the wound-site adhesive layer 20, 220 such that an adjacent upper layer, such as the backing layer 12 and/or the wound contact layer 18 may be exposed through the perforations 22 whilst being adherable to the wound site via the adhesive layer 20, 220.

The perforations 22 may be any suitable shape and may be provided in any suitable pattern or distribution. In some embodiments, the shape of perforations 22 may be round, e.g. circular or oval. Providing round perforations may be advantageous for manufacturing purposes. In particular, when the perforations are formed with a punch, a round hole is less likely to result in a residual attachment of the cut-out portion (which may be referred to as a slug) when compared to a shape in which the cut out has an acute apex or corner feature. However, it will be appreciated that the size and shape of the perforations 22 may differ from one another.

The distribution of the perforations 22 may be region specific or continuous across the extent of the wound adhesive layer 20. The distribution, and optionally size and optionally shape, may be uniform across a given region of the wound dressing. For example, the perforations 22 may be equidistantly spaced having a first distance between adjacent perforations 22 in a border region and equidistantly spaced by a second distance across the wound contact layer 18. In other embodiments, the perforations 22 may be uniformly distributed across the full extent of the wound-site adhesive layer 18.

Figure 3 shows a schematic representation of a wound-site adhesive layer 320 comprising a distribution of perforations 22 provided in a uniform array. The perforations 22 are provided along intersecting lines L1-L4 which form a diamond lattice across the extent of the adhesive layer 320, when viewed in plan. Each cell LC of the diamond lattice, of which only one is highlighted, may comprise a perforation 22 at each apex and have a major axis and a minor axis in which the major axis is longer than the minor axis. In the embodiment shown in Figure 3, the major axis extends horizontally and the minor axis extends vertically, but this is not determinative and does not indicate an orientation associated with how the wound dressing 10, 210 might be applied. The perforated adhesive layer 320 shown in Figure 3 is provided with a curved peripheral edge, however, this is not a limitation and the wound dressing 10, 210 and wound-site adhesive layer 22 may be any shape.

To provide the lattice arrangement of perforations 22, the perforations 22 may be arranged in a plurality of equidistantly spaced rows Rᵢ and a plurality of equidistantly spaced columns Cⱼ, where i and j are integers. The rows Rᵢ and columns Cⱼ each comprise a linear array of equidistantly spaced perforations 22. Adjacent columns Cⱼ are axially offset (with respect to the longitudinal axis of the column) from one another so such that the perforations 22 from the odd numbered columns form a first row R₁, and the perforations 22 from the even number columns form a second row R₂ which is offset and arranged along a separate line from the first row R₁. The spacing between the perforations 22 in the columns Cⱼ may be smaller than the spacing of the perforations in the rows Rᵢ.

The spacing between the centres of the columns Cⱼ may be greater than the spacing between the rows Rᵢ. The spacing of the columns Cⱼ may be between 3.9mm and 8.1mm, with the spacing of the rows Rᵢ being between 2.5mm and 5.1mm. When considered as the aforementioned diamond lattice, the lines of the lattice may be inclined to the horizontal as indicated by α.

The wound-site adhesive layer 20, 220, may be provided as a separate layer which is laminated to the wound dressing during the manufacturing process. The manufacturing of the perforations 22 may be achieved using any suitable known technique. One conventional technique includes punching holes in an adhesive sheet prior to it being laminated to the wound dressing 10, 210. Another technique includes laser cutting of the perforations.

As noted above, in some embodiments, a wound dressing 10, 210 may include a uniform distribution of similar sized perforations in the wound contact layer 18 region, and a different size or no perforations in the peripheral region of the dressing 10, 210. Similarly, a uniform distribution of perforations may be provided on the peripheral region when the central wound contact layer regions is adhesive free.

However, the manufacturing of a wound-site adhesive layer 20 with a nonuniform or local specific distribution/size/shape requires a more involved manufacturing process. Manufacturing an adhesive layer 320 may be more straightforward and inexpensive when the perforations 22 of a common size and distribution are provided such that the adhesive layer can be provided in larger sheets or rolls and can be subsequently laminated together with the other layers in the dressing 10, 210. However, providing a uniform distribution of perforations 22, in terms of size, shape and/or layout, may be detrimental to the performance requirements discussed above in terms of controlling the exudate absorption rate and wear time.

Returning to Figures 1 and 2, as noted above, the coat weight of the wound-site adhesive layer 20, 220 may be between 70gsm and 200gsm. For example, the base weight may be 70gsm, 80gsm, 100gsm, 125gsm, 130gsm, 150gsm, or 200gsm. Generally, in the prior art there is a trend between adhesive coat weight and adhesive strength, however the applicants have discovered that for different formulations of adhesive a plateau at which increases of adhesion will be achieved through increasing of coat weight will vary. Additionally increasing coat weight of the adhesive, in turn increases thickness which increases potential for the adhesive edge of the wound dressing to mechanically deform. i.e. tuck or roll up. The applicants have found that a coat weight towards the lower end of the range, for example, 70-100gsm, such as 70gsm provides a reasonable balance between adhesion performance and thickness to reduce internal mechanical stresses in the wound dressing.

Although not shown in the drawings, the wound dressing may comprise a release layer which covers and preserves sterility of the wound-site adhesive layer 20 prior to use and during the application to a wound site, as well known in the art.

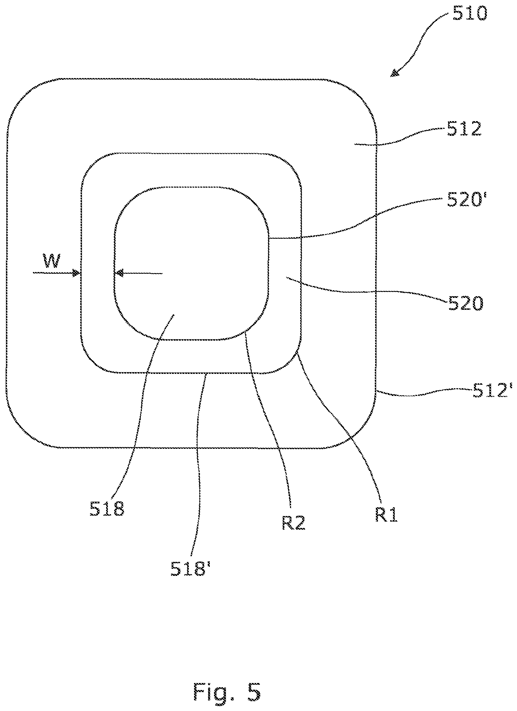

As noted above in connection with Figure 1, the border region of wound-site adhesive layer may partially overlap the wound contact layer 18 such that the wound contact layer 18 is held in a fixed relation to the backing layer 12 via the adhesive layer. The overlap may comprise a width which is defined as the distance between the outer edge 18a of the wound contact layer 18 and the inner edge 16a of the wound-site adhesive layer 20. In some embodiments, the extent of the overlap may be broadly similar around the periphery of the wound contact layer 18 such that the overlap has a constant width. In other embodiments, the width of the overlap may vary around the periphery. In some embodiments, the dressing may be polygonal, e.g. quadrilateral/rectangular/square, when viewed in plan and the overlap may be include one or more corner regions. The corner regions may be defined in part by a radius of curvature. As shown in Figure 5, in some embodiments, the radius of curvature of the adhesive layer may be greater than the radius of curvature of the wound contact layer which results in the overlap being greater in the corner regions.

Figure 5 shows a dressing 500 having a backing layer 512, a wound contact layer 518, and an adhesive layer 520. The backing layer 512 is defined by an outer peripheral edge 512' and extends across the full width of the dressing. The adhesive layer 520 extends from the outer peripheral edge of the backing layer 512' to an inner edge 520' to provide a central region in which the wound contact layer 518 is exposed. The wound contact layer 518 includes outer edges 518'.

The dressing is generally square when viewed in plan with the wound contact layer edge 518' comprising a first radius, R1, which is smaller than the inner edge radius R2 of the adhesive layer. The relative difference in the corner radiuses R1 and R2 allows the contacting area between the wound contact layer 518 and adhesive layer 520 to be increased when compared to dressings where the radius of curvature is the same. Increasing the overlap at the corner regions may allow for a stronger attachment between the wound contact layer 18 and the adhesive layer 20 without significant detriment to the absorbency of the dressing.

In the embodiment shown in Figure 5, the dressing 510 may comprise an 85mm by 85mm (+/-5mm) backing layer when viewed in plan. The radius of curvature of the adhesive layer edge 20' may be between 10mm and 15mm, preferably around 12.5mm. The radius of curvature of the wound contact layer edge 518' may be between 7.5mm and 12.5mm, preferably around 10mm, but in any case, less than the radius of curvature of the adhesive layer edge 520'. The width of the overlap away from the corner regions may be constant. In the embodiment shown in Figure 5, the width of the overlap, w, is between 10mm and 20mm, preferably around 15mm. The width of the wound contact layer exposing aperture in adhesive layer may be between 30mm and 40mm. The width of the wound contact layer may be between 45mm and 50mm.

The increased curvature of the inner edge corners of the adhesive layer may provide an increase of approximately 3-6% when compared to dressings in which the radius of curvature is constant.

Although the dimensions given above relate to a specific embodiment, it will be appreciated that the ratios of the various measurements may be applied to other dressings. Further, although the dressing is shown as having substantially straight sides, the sides may include a general curvature whilst still being distinguishable from the corner regions.

As noted above, some embodiments may comprise a super-absorbency layer 14, such as those shown in Figures 1 and 2. The wound contact layer 18 may comprise a first surface for directly contacting a wound bed and a second surface which faces the backing layer 12. As shown in Figures 1 and 2, the superabsorbent layer 14 may be located between the backing layer 12 and the second surface of the wound contact layer 18.

The wound contact layer 18 may comprise gel forming fibres. As noted in the background section, dressings which comprise absorbents that gel are known in the art and manage exudate by absorbing it and retaining it within the dressing. The moisture in the gelling absorbent can still be lost by vapour transmission from the dressing, but less readily than with a foam absorbent because the exudate is retained and held within the gelled absorbent. As the absorbent is acting as a reservoir for exudate, it needs to have sufficient capacity to retain exudate throughout the wear time of the dressing. This affects the quantity of absorbent needed in the wound dressing which of course affects the thickness and conformability of the dressing overall. In addition, in general with gelling absorbents, particularly fibrous gelling absorbents, the relationship between the thickness of the gelling absorbent layer or its weight per unit area and its absorbency is not linear. This means that, in conventional gelling absorbents, a trade-off is required between absorbency, conformability and moisture vapour transmission.

The applicant's granted patent EP2498829 provides an absorbent component for a wound dressing having a wound contacting layer comprising gel forming fibres bound to a foam layer so as to provide a combined use of a wound contact layer which absorbs exudate by gelling so that lateral spread of the exudate is contained, with a foam layer which absorbs exudate but readily releases it through moisture vapour transmission. The presence of the gel forming wound contact layer was considered at the priority date of EP2498829 to limit the lateral spread of exudate in the foam and increase its exudate retention compared to the use of foam alone and improved the moisture vapour transmission properties of the wound contacting layer.

The present invention provides a development of the concept described in EP2498829 in which, in place of a foam layer which absorbs the exudate and releases it through moisture vapour transmission, a super-absorbency layer is provided in conjunction with the foam layer. The applicants have discovered that, in place of using a foam layer to absorb the exudate and release it through moisture vapour transmission, the use of a super-absorbency material to absorb and retain the exudate can improve exudate removal rates and wear times for a wound dressing, whilst limiting lateral spread of the exudate. Thus, the present invention provides a dressing which increases the fluid handling capacity and retention whilst preventing lateral spread. As such, the use of a super-absorbency layer in conjunction with the gelling absorbent allows a greater amount of fluid to be locked away without a reliance on moisture vapour release from the dressing. Although this generally leads to an improved dressing, this may be particularly advantageous when the dressing is covered, for example, a compression bandage is used in conjunction with the dressing and MVTR is reduced.

Super-absorbent polymers are known in the art for their fluid absorption and retention properties and are utilised in across different industries. Super-absorbency layers are generally available in two forms: super absorbent (e.g. polyacrylate) fibres, SAF, and super absorbent (e.g. polyacrylate) polymers, SAP. Wound dressings of the present disclosure may be provided with a super-absorbency layer comprising either or both of SAF and SAP, however, in preferred embodiments, the super-absorbency layer comprises SAF only. Providing a SAF only super-absorbency layer allows the wound dressing to be cut prior to use which is not possible with a SAP or SAF/SAP blend due to the particulate nature of the SAP. Further, the use of a SAF only super-absorbency layer has been found to allow for improved conformity when used in a laminated dressing, particularly where intra-layer adhesives are used, as described below.

The use of SAF also provides increased fluid retention performance when compared to SAP and a dressing with no super-absorbent layer. Table 1 below provides comparative data for a dressing comprising a prior art gelling absorbent/foam dressing combination (sample 1), and prototype dressings comprising a gelling wound contact layer combined with: i) an unrestricted SAP layer in which the wound contact layer and super-absorbency are free to move relative to one another (sample 2), ii) a laminated gelling wound contact layer and SAP layer, and iii) a laminate gelling wound contact layer and SAF layer.

**Table 1 showing fluid retention properties of a dressing with no super-absorbency layer and various SAP and SAF layers.**

| Sample number | Sample type | Fluid Absorbed per gram of sample (g/g) | Fluid Absorbed per unit area (g/cm²) | Fluid Retained per gram of sample (g/g) | Fluid Retained per unit area (g/cm²) | % Retained |
|---|---|---|---|---|---|---|
| 1 (Prior art) | Gelling absorbent | 11.13 | 0.45 | 8.88 | 0.36 | 79% |
| 2 | Unrestricted SAP | 9.43 | 0.99 | 6.58 | 0.69 | 70% |
| 3 | Laminated SAP | 20.55 | 0.77 | 13.86 | 0.52 | 67% |
| 4 | Laminated SAF | 13.39 | 0.89 | 11.66 | 0.77 | 87% |

As can be seen from Table 1, the use of a laminated SAF may provide an increased level of absorbency per unit area when compared to a laminated SAP product and increased fluid retention.

In preferred embodiments, the super-absorbency layer may comprise a blend of sodium polyacrylate and polyester fibres. The ratio of the super absorbent and polyester fibres may be between 40:60 to 55:45. In some preferred embodiments, the super absorbency layer may comprise a 40:60 ratio. Providing a lower amount of the super absorbent fibres may prevent distortion of the layer dressing during subsequent manufacturing steps, in particular during sterilisation of the dressing, with limited impact on the conformity or fluid handling capability of the dressing.

The super absorbency layer may comprise non-woven fibres and have a thickness of between 1.5mm and 2.5mm. The thickness may optionally be 2mm in some preferred embodiments. The thickness of the super-absorbent layer may be determined from a balance of patient comfort, conformability, and fluid handling performance.

As noted above, in addition to the super-absorbency layer 14 and wound contact layer 18, the wound dressing 10, 210 may comprise a foam layer 16. The foam layer 16 may be provided to improve the comfort level and tacticity of the wound dressing. As noted above, the foam layer 16 may be provided between the wound contact layer 18 and super-absorbency layer 14, or between the super-absorbency layer 14 and backing layer 12.

The foam layer 16 is preferably a hydrophilic foam such as polyurethane and more preferably is a hydrophilic open celled foam such as those available fromFreudenberg, or Filtrona and in particular Filtrona 30W . The foam typically has a thickness of 0.25mm to 5mm, preferably from 1mm to 4.0 mm and most preferably from 1.5mm to 3mm. The foam layer preferably has an absorbency of 10 to 20 g/g when measured by the free swell absorbency method (BS EN 13726-1 :2002).

The foam layer may have a thickness of between 1.3mm and 3.2mm, optionally 2.5mm. The foam may be polyurethane, PU. In one advantageous embodiment an aliphatic polyurethane foam may be 1.5mm or 3mm thick. In another advantageous embodiment, the MDI polyurethane foam may be 1.5mm or 3mm thick.

To demonstrate the effectiveness of the gelling wound contact layer and super-absorbency layer, six prototype dressings were created. Each of the dressings comprises a ConvaTec^{®} Hydrofibre^{®} wound contact layer having 150gsm base weight and a polyurethane 30 micron backing layer. In addition to the wound contact layer and backing layer, each of the prototypes included either a 2.5mm PU foam or a 1.5mm MDI foam. Prototypes 1 and 2 did not include a super-absorbency layer. Prototypes 3 and 4 were provided with the SAF layer immediately below the backing layer followed by the respective foam layer and wound contact layer. Prototypes 5 and 6 were provided with the foam layer immediately below the backing layer followed by the wound contact layer. Thus, the order of the foam and SAF layers were interchanged in prototypes 3 and 4, and 5 and 6. Five samples were tested for each prototype.

Table 2 shows fluid absorbed by the prototype wound dressings and the fluid retained, the percentage retention. The final two columns indicate which foam was used (X indicating the use of the foam).

**Table 2 showing a comparison of absorbency and retention for dressings without a SAF layer, and with a SAF layer.**

| Prototype | Fluid absorbed per unit area (g/cm²) | Fluid retained per unit area (g/cm²) | % Retention | Foam 2.5mm PU | Foam 1.5mm MDI |
|---|---|---|---|---|---|
| 1 - no SAF | 0.5 | 0.43 | 81 | X | |
| 2 - no SAF | 0.4 | 0.36 | 91 | | X |
| 3 - Upper Foam | 0.79 | 0.66 | 85 | X | |
| 4 - Upper Foam | 0.67 | 0.61 | 91 | | X |
| 5 - Upper SAF | 0.83 | 0.71 | 86 | X | |
| 6 - Upper SAF | 0.8 | 0.72 | 90 | | X |

As can be seen from the data provided in Table 2, the dressings all show a large percentage retention, with the super-absorbency layer providing an increase in absorbency where present. The positioning of the foam has limited impact on the retention. The thicker 2.5mm PU foam provides an increase in absorbency but and the MDI foam provides increased retention. Increasing the thickness of the foam layer further, for example to 3mm, may increase the absorbency further. However, increasing the foam thickness has been shown to reduce the percentage retention, as is generally indicated by the 2.5mm PU foam having the lower retention vis-à-vis the thinner 1.5mm MDI foam.

As noted above, the wound contacting layer is fibrous and comprises gel-forming fibres. Fibrous layers, as opposed to polymeric layers, have the advantage that they are especially able to gel block which resists the lateral spread of exudate. In addition, exudate is absorbed rapidly and is retained under pressure.

The fibres suitable for use in the wound contact layer of the present disclosure include hydrophilic fibres, which upon the uptake of wound exudate become moist and slippery and gelatinous and thus reduce the tendency of the surrounding fibres to stick to the wound. The fibres can be of the type which retain their structural integrity on absorption of exudate or can be of the type which lose their fibrous form and become a structureless gel or a solution upon absorption of exudate.

The fibres are preferably chemically modified cellulose fibres and in particular spun sodium carboxymethylcellulose fibres or cellulose ethyl sulphonate fibres. The carboxymethylcellulose fibres are preferably as described in PCT WO/9312275 or GB93/01258. The fibres may also be pectin fibres, alginate fibres and particularly those as described in WO94/17227 or EP433354 or EP476756 or composite fibres of alginate and polysaccharide such as those described in EP 0892863, chitosan fibres, hyaluronic acid fibres, or other polysaccharide fibres or fibres derived from gums . The cellulosic fibres preferably have a degree of substitution of at least 0.05 carboxymethyl groups per cellulose unit. Preferably the gel forming fibres for use in the present invention have an absorbency of either water or saline of at least 15g/g as measured by the free swell absorbency method, more preferably at least 25g/g or 50g/g. The degree of substitution of the carboxymethylated cellulose gel forming fibres is preferably at least 0.2 carboxymethyl groups per cellulose unit, more particularly between 0.3 and 0.5.

The gel forming fibres are preferably mixed to give a wound contacting layer comprising fibres of different absorbencies or properties. The wound contacting layer may comprise other fibres such as textile fibres which can be natural or synthetic, but are preferably cellulosic fibres for example viscose rayon, multi-limbed viscose, cotton or regenerated cellulose having a higher absorbency than most textile fibres. Textile fibres typically have an absorbency of less than lg/g when measured by the free swell absorbency test. The wound contacting layer can be made from a non-woven, fibrous web formed by any of the following methods: needle punched, spunlaced, wet- laid, dry laid, meltblown or felted. The web can then be stitch bonded with strengthening fibres or yarns to provide additional strength to the layer such that it retains its structure when saturated with exudate. Additionally, the stitchbonded structure may afford higher absorbency or a degree of extensibility to the dressing depending on the nature of the strengthening fibres and yarns used and their stitchbonding pattern. The wound contact layer is preferably between 20 microns and 5mm thick, more preferably 2mm to 3mm thick and even more preferably from 1mm to 2mm thick. In some embodiments, the thickness may be 1mm or 1.5mm, for example.

The base weight of the wound contact layer may affect the fluid handling capacity of the wound dressing, typically with an increase in the base weight beneficially increasing the absorbency. Surprisingly, the applicants have found that reducing the base weight in a dressing comprising a super-absorbency layer, in particular, a SAF super-absorbency layer, has minimal impact on the fluid absorbency and fluid retention. To demonstrate this, four prototypes of wound dressing were trialled to determine the level of the base weight for use with the super-absorbency layer. The four prototypes, as shown in Table 4. include a first wound dressing comprising a 100gsm wound contact layer with no supcr-absorbcncy layer, a second wound dressing comprising a 70gsm wound contact layer, a 2.5mm polyurethane foam and a 2mm SAF super-absorbency layer, a third wound dressing with 100gsm SAF wound contact layer, a 2.5mm polyurethane foam and a 2mm SAF super-absorbency layer, and a fourth wound dressing comprising 70gsm wound contact layer, a 2.5mm polyurethane foam and a 2mm SAF super-absorbency layer. The wound contact layers were Hydrofiber^{®} from ConvaTec. The first sample was a prior art Aquacel^{®} Foam product comprising a 100gsm Hydrofiber^{®} layer bonded to a PU foam. Table 4 below summarises the results of a fluid absorption and retention test for n=5.

**Table 4 - Fluid Absorption and Retention of various wound dressings with no super-absorbency layer and with an SAF super-absorbency layer having different wound contact base layers.**

| Sample | Absorbency | Retention | % Retention |
|---|---|---|---|
| 100gsm Aquacel^{®} foam | 0.41 | 0.26 | 63 |
| 70gsm Hydrofiber^{®}. 2.5mm foam, 2mm SAF | 0.91 | 0.75 | 82 |
| 100gsm Hydrofiber^{®}, 2.5mm foam, 2mm SAF | 0.89 | 0.77 | 87 |
| 150gsm Hydrofiber^{®}, 2.5mm foam, 2mm SAF | 0.91 | 0.79 | 86 |

As can be seen, the increase in base weight has minimal impact on the absorbency and retention of the wound dressing when used with a SAF super-absorbency layer in combination with a foam. However, providing a lower base weight can help reduce the interlaminar stress and associated deformation of the wound dressing and so the 70gsm wound contact layer may be used in some preferred embodiments. Further, the lower base weight may result in a thinner dressing. 70gsm may also represent a sensible minimum for manufacturing due to the lack of strength in lower weights.

As noted above in connection with Figures 1 and 2, the dressing may comprise a backing layer in which the wound contact layer and superabsorbent layer are provided. The backing layer may be breathable so as to allow moisture vapour release from the dressing whilst preventing ingress of contaminants to the wound site. The backing layer may be any suitable material and may, in some embodiments, comprise a polyurethane material, as known in the art. The backing layer may comprise a blown polyurethane or a solvent cast polyurethane, the latter of which may reduce in use deformation and delamination. In some preferred embodiments, the backing layer may be Convestro 9147 supplied by Covestro AG or Transcontinental Coartings Inspire 2301. The backing layer may have a thickness of between 25 microns and 35 microns, preferably 30 microns. Preferably the film layer has an MVTR (liquid in contact) of at least 10,000/m2/24hrs.

In some embodiments the backing layer may be adhesive or include an adhesive film to provide the wound site adhesive. The adhesive may extend across the full extent of the backing layer such that the backing layer adheres at a border of the dressing around the wound site, and also to the upper most layer in the dressing stack, for example, the super-absorbent layer. However, as described below, in some embodiments, the backing layer may be adhered to the skin alone, with the central region adjacent to which the upper layer of the dressing is located and contacted is adhesive free. It will be appreciated that the wound-site adhesive may be applied directly to the backing layer, or may be a separate sheet which is laminated to the backing layer.

The adhesive or adhesive film may comprise an acrylic adhesive as known in the art. The acrylic adhesive may have a 5-20 g/m² and may be comprise a hexagonal pattern. The pattern of the adhesive may be selected to increase the open area to and breathability whilst providing sufficient surface area to stick to the contacting material. It will be appreciated that the lines of adhesive define hexagonal gaps so as not to create complete open channels within the layer.

As noted below, the wound dressing may comprise a laminated structure in which the various layers are bonded together to prevent in use and pre-application movement. Bonding the various layers to one another may be achieved using any suitable technique such as a polymer based melt layer, by an adhesive, by flame lamination, by direct lamination via foam casting, or by ultrasound. Additionally or alternatively the intra-layer adhesives 24, 26, as shown in Figure 4, may used to bond the various layers together.

The intra-layer adhesive (which may be referred to as a binder) may be a continual later, perforated layer, liquid or scatter coat adhesive for example. The binder may be a web comprising a continuous or broken layer of adhesive which is inserted in between the respective adjacent layers and activated to bond the layers together. The binder may be thermo activated for example in which heat and pressure are applied to during a lamination process. The film may comprise a web of adhesive strands or dots.

Scatter coat adhesives are known in art and relate to a method of providing a layer or adhesive by distributing a powdered adhesive onto a surface prior to laminating the layers. The scatter coat adhesive may be used for some or all of the intra-layer adhesives. For example, the scatter coat adhesive may be used to adhere the super-absorbent layer to the foam and the foam may be adhered to the wound contact layer using an alternative bonding technique.

In some embodiments, some of the adjacent layers may not include intralayer adhesives. For example, as shown in Figures 1 and 2. the upper layer of the stack, e.g. the super-absorbency layer, may not be bonded to the backing layer such that the backing layer and upper layer are free to move relative to one another. Hence, one or more internal layers of the wound dressing may be provided with an adhesive free region between the second surface of the super-absorbent layer and the backing layer in which there is no adhesive. When this is the case, the wound dressing may include an internal stack of layers including, for example, a wound contact layer, a foam layer and a super-absorbent layer, that are attached to the backing layer via the wound contact layer. As noted above, the wound contact layer may be attached to the backing layer via a wound-site contact layer.

In this way, the internal stack is configured to be moveable relative to the backing layer during manufacture and use. In particular, the separation between the internal stack, or at least an upper layer thereof, and the backing layer, allows for the relative contraction or expansion of the respective layers during manufacture of the dressing. This may allow for alternatively and advantageous operations to be conducted during the manufacture of the dressing.

Once such manufacturing step may include sterilisation of the dressing. In some embodiments, the wound dressings described herein may be sterilised using an ethylene oxide technique. However, using an ethylene oxide sterilisation may result in the relative movement of one or more layer of the internal stack which, if adhered to the backing layer, would cause a deformation, e.g. wrinkling or warping, of the backing layer resulting in an unsightly appearance. Hence, providing some relative separation between the layers may be advantageous.

The one or more embodiments are described above by way of example only. Many variations are possible without departing from the scope of protection afforded by the appended claims.

The invention may be defined by the following clauses:
1. A wound dressing comprising:
   a backing layer;
   a wound contact layer having a first surface for directly contacting a wound bed and a second surface facing the backing layer, the wound contact layer comprising gel forming fibers; and,
   a superabsorbent layer, wherein the superabsorbent layer is located between the backing layer and the second surface of the wound contact layer.
2. The wound dressing of clause 1, wherein the superabsorbent layer has an outer profile and the wound contact layer has a corresponding outer profile which is in register with the outer profile of the superabsorbent layer.
3. The wound dressing of clause 1 or 2, further comprising: a foam layer located between the backing layer and the second surface of the wound contact layer.
4. The wound dressing of clause 3 when clause 3 is dependent on clause 2, wherein the foam layer has a foam layer outer profile which corresponds with and is in register with the outer profile of the superabsorbent layer.
5. The wound dressing of clause 3 or 4, wherein the foam layer is located between the backing layer and the superabsorbent layer.
6. The wound dressing of any preceding clause, wherein the superabsorbent layer comprises fibers, optionally non-woven fibers.
7. The wound dressing of clause 6, wherein the superabsorbent layer comprises non-woven fibers only.
8. The wound dressing of clause 7, wherein the non-woven fibers are polyacrylate fibers.
9. The wound dressing of any preceding clause, wherein the superabsorbent layer has a thickness of between 1.5mm and 2.5mm , optionally 2mm.
10. The wound dressing of any preceding clause, wherein the superabsorbent layer comprises a first surface facing the wound contact layer and a second surface facing the backing layer, the superabsorbent layer first surface comprising an intra-layer adhesive.
11. The wound dressing of clause 10, wherein the super-absorbent layer second surface is configured to be movable relative to the backing layer.
12. The wound dressing of clause 11, wherein the superabsorbent layer contacts the backing layer and wherein the backing layer comprises an adhesive border region which surrounds a non-adhesive central region in which the superabsorbent layer is located fully within such that the superabsorbent layer is not adhered to the backing layer.
13. The wound dressing of any preceding clause, further comprising a wound-site adhesive layer for adhering the wound dressing at a wound site, wherein the adhesive layer comprises a border region which surrounds the wound contact layer.
14. The wound dressing of clause 13, wherein the adhesive layer extends continuously across the first surface of the wound contact layer.
15. The wound dressing of either of clause 13 or 14, wherein the adhesive layer comprises perforations, the perforations having a diameter of between 1.6mm and 2.7mm, optionally 2.2mm.
16. The wound dressing of any of clause 12 to 15, wherein the adhesive layer comprises a silicone adhesive.
17. The wound dressing of any of clause 13 to 16, wherein the adhesive layer comprises coat weight of 70gsm to 150gsm.
18. The wound dressing of any of clause 3 to 17, wherein the foam layer comprises a first, wound facing surface and a second, backing layer facing surface, the foam layer first surface comprising a foam first adhesive layer.
19. The wound dressing of clause 18, wherein the second surface comprises a foam second adhesive layer.
20. The wound dressing of clause 19, wherein the foam layer and superabsorbent layer are adhered together.
21. The wound dressing of any of clause 3 to 20, wherein the foam layer is an aliphatic foam or a methylene diphenyl disoocyanate foam.
22. The wound dressing of any of clause 3 to 21, wherein the foam is a polyurethane foam.
23. The wound dressing of any of clause 3 to 22, wherein the foam layer has a thickness of between 1.3mm and 3.2mm, optionally 2.5mm.
24. The wound dressing of any preceding clause, wherein the wound contact layer comprises a cellulose fiber.
25. The wound dressing of clause 24, wherein the wound contact layer comprises carboxymethylated cellulose fibers.
26. The wound dressing of any preceding clause, wherein the wound contact layer has a thickness between 1mm and 1.5mm.
27. The wound dressing of any preceding clause, wherein the wound contact layer has a base weight of between 70gsm and 150gsm, optionally 70gsm.
28. The wound dressing of any preceding clause, wherein the backing layer comprises a polyurethane material.
29. The wound dressing of any preceding clause, wherein the backing layer has a thickness of 30 microns.

## Claims

1. A wound dressing comprising:
a backing layer;
a wound contact layer having a first surface for directly contacting a wound bed and a second surface facing the backing layer; and
a superabsorbent layer, wherein the superabsorbent layer comprises a blend of sodium polyacrylate fibres and polyester fibres.

2. The wound dressing of claim 1, wherein the ratio of the superabsorbent and polyester fibres is between 40:60 to 55:45.

3. The wound dressing of claim 2, wherein the superabsorbent layer comprises a 40:60 ratio.

4. The wound dressing of any preceding claim, wherein the superabsorbent layer is located between the backing layer and the second surface of the wound contact layer.

5. The wound dressing of any preceding claim, further comprising a foam layer located between the backing layer and the second surface of the wound contact layer.

6. The wound dressing of claim 5, wherein the foam layer comprises a polyurethane foam.

7. The wound dressing of claim 5 or 6, wherein the foam layer comprises an aliphatic foam or a methylene diphenyl disoocyanate foam.

8. The wound dressing of any of claims 5 to 7, wherein the foam layer has a thickness of between 1.3mm and 3.2mm, optionally 2.5mm.

9. The wound dressing of any preceding claim, wherein the superabsorbent layer comprises a first surface facing the wound contact layer and a second surface facing the backing layer.

10. The wound dressing of claim 9, wherein the superabsorbent layer first surface comprises an intra-layer adhesive.

11. The wound dressing of claim 10, wherein the intra-layer adhesive is a continual layer, perforated layer, liquid or scatter coat adhesive.

12. The wound dressing of any of claims 9 to 11, wherein the superabsorbent layer second surface is configured to be movable relative to the backing layer.

13. The wound dressing of any preceding claim, wherein the superabsorbent layer has a thickness of between 1.5mm and 2.5mm, optionally 2mm.

14. The wound dressing of any preceding claim, wherein the backing layer comprises a polyurethane material.

15. The wound dressing of any preceding claim, wherein the backing layer has a thickness of 30 microns.
